# FASCICULE DE BREVET EUROPEEN

(11) **EP 1 532 966 B1**
(45) Date de publication et mention de la délivrance du brevet: **02.01.2008**
(21) Numéro de dépôt: 04292635.2
(22) Date de dépôt: 08.11.2004
(51) Int. Cl.: A61K 8/73, A61K 8/20, A61K 8/81, A61K 8/34, A61Q 5/06, A61Q 5/00

(54) **Composition cosmétique comprenant de la gomme de gellane ou un de ses dérivés, un polymère fixant, un sel monovalent et un alcool, procédés mettant en oeuvre cette composition et utilisations**
Kosmetische Zusammensetzung enthaltend Gellangummi oder ein Derivat davon, ein fixierendes Polymer, ein einwertiges Salz und ein Alkohol, Verfahren auf Basis dieser Zusammensetzung und Anwendungen
Cosmetic composition comprising gellan gum or a derivative thereof, a fixing polymer, a monovalent salt and an alcohol, processes using this composition and uses thereof

(30) Priorité: 18.11.2003 FR 0313486
(43) Date de publication de la demande: 25.05.2005
(73) Titulaire: L'ORÉAL, 75008 Paris (FR)
(72) Inventeur: Marie, Laurence, 93200 Saint-Denis (FR); Laurent, Ludivine, 92400 Courbevoie (FR); Pasquet, Dorothée, 92270 Bois-Colombes (FR); Breton, Marie-Laure, 75011 Paris (FR)
(74) Mandataire: Dossmann, Gérard

(56) Documents cités:
- EP-A- 0 985 410
- EP-A- 1 516 613
- US-A- 6 126 930
- US-B2- 6 624 125

## Description

La présente demande a pour objet une composition cosmétique liquide, aqueuse, comprenant, dans un milieu cosmétiquement acceptable, au moins une gomme de gellane ou un de ses dérivés, un polymère fixant, un sel monovalent, et un alcool. La présente demande concerne encore un procédé pour la mise en forme ou le maintien de la coiffure dans lequel cette composition cosmétique est mise en oeuvre, ainsi que les utilisations de cette composition.

Les compositions cosmétiques pour la mise en forme et/ou le maintien de la coiffure les plus répandues sur le marché de la cosmétique sont des compositions à pulvériser essentiellement constituées d'une solution le plus souvent alcoolique et d'un ou plusieurs composants, appelés composants fixants, qui sont généralement des résines polymères dont la fonction est de former des soudures entre les cheveux. Ces composants fixants sont souvent formulés en mélange avec divers adjuvants cosmétiques.

Ces compositions cosmétiques sont généralement conditionnées soit dans un flacon pompe, soit dans un récipient aérosol approprié mis sous pression à l'aide d'un agent propulseur, le système aérosol contient alors d'une part une phase liquide (ou jus) et d'autre part d'un agent propulseur.

Une fois appliquée sur les cheveux, la phase liquide, contenant les composés fixants et un solvant approprié, sèche permettant la formation de soudures nécessaires à la fixation de la chevelure par les composants fixants. Les soudures doivent être suffisamment rigides pour assurer le maintien des cheveux, cependant elles doivent être également suffisamment fragiles pour que l'utilisateur puisse, en peignant ou en brossant la chevelure, les détruire sans heurter le cuir chevelu ni endommager les cheveux.

Les résines filmogènes classiques généralement utilisées en tant qu'agent fixant en milieu alcoolique présentent l'inconvénient de conférer de médiocres propriétés cosmétiques à la composition coiffante, en particulier le toucher obtenu par la mise en oeuvre des compositions à base de résines filmogènes n'est pas très satisfaisant.

Pour améliorer le toucher de ces compositions, on a souvent eu recours, dans l'art antérieur à un ajout de silicones, cependant cette solution ne permet pas d'améliorer les propriétés cosmétiques dans le cas des compositions hydro-alcooliques

Le document brevet EP-A-0 985 410 (SHISEIDO CO LTD) 15 mars 2000 (2000-03-15) décrit (ex. 6) une composition cosmétique liquide, aqueuse, contenant, dans un milieu cosmétiquement acceptable au moins une gomme de gellane, un polymère épaississant (Carbopol 1342), au moins un sel de cation monovalent (KOH) et au moins un alcanol en C₁-C₄ (ethanol).

Le document brevet EP-A-1 516 613, opposable uniquement au titre de l'Article 54(3) CBE, décrit (ex. 5.3) une composition cosmétique liquide, aqueuse, contenant, dans un milieu cosmétiquement acceptable au moins une gomme de gellane, au moins un polymère fixant (PVP/VA), au moins un sel de cation monovalent (sodium benzoate/salicylate) et au moins un alcanol en C₁-C₄ (ethanol).

De manière surprenante est avantageuse, la demanderesse a découvert que l'utilisation d'une combinaison d'une gomme de gellane ou un de ses dérivés, d'un polymère fixant, d'un sel monovalent et d'un alcool permet d'améliorer les propriétés cosmétiques de ces compositions.

Les compositions selon la présente invention permettent une bonne fixation et un bon maintien des cheveux c'est-à-dire un effet coiffant qui persiste tout au long de la journée, voire plusieurs jours, qui présente une bonne tenue à l'humidité et qui s'élimine facilement au shampooing.

Ces compositions permettent aussi de conférer de bonnes propriétés cosmétiques aux cheveux notamment en termes de toucher (toucher doux) et de démêlage.

Il a également été observé que la combinaison selon l'invention permet une parfaite mise en suspension des particules solides telles que les paillettes et que le caractère thixotrope de cette combinaison permet une restitution parfaite de la composition cosmétique coiffante lorsqu'elle est appliquée au moyen d'un spray ou en aérosol.

La présente invention a pour objet une composition cosmétique liquide, aqueuse, contenant, dans un milieu cosmétiquement acceptable au moins une gomme de gellane ou un de ses dérivés, au moins un polymère fixant, au moins un sel de cation monovalent, le contre-ion étant un anion minéral, et au moins un alcool choisi parmi les alcanols en C1-C4.

De préférence, la composition cosmétique selon l'invention est une composition cosmétique capillaire, de manière préférée une composition cosmétique capillaire coiffante.

Un autre objet de la présente invention consiste en un procédé pour la mise en forme ou le maintien de la coiffure dans lequel la composition cosmétique selon l'invention est mise en oeuvre.

Un troisième objet de l'invention concerne les utilisations de cette composition cosmétique en tant que composition coiffante pour la fixation et le maintien des cheveux, composition de soin des cheveux, composition de conditionnement des cheveux notamment pour apporter de la douceur aux cheveux, ou encore composition de maquillage des cheveux.

Ainsi, la présente demande concerne l'utilisation de la composition cosmétique selon l'invention pour apporter de la fixation aux cheveux, pour apporter du maintien aux cheveux, pour apporter de la cosmétique aux cheveux.

La composition cosmétique selon l'invention peut se présenter sous la forme d'un spray, d'une mousse ou d'un gel.

D'autres objets, caractéristiques, aspects et avantages de l'invention apparaîtront encore plus clairement à la lecture de la description et des exemples qui suivent.

Sans vouloir être lié à une quelconque théorie, les compositions cosmétiques selon la présente demande peuvent se présenter sous forme de gels, c'est-à-dire un réseau tridimensionnel de molécules qui retient dans ses mailles une quantité importante de solvant. La formation d'un tel réseau constitue sa gélification.

Les compositions cosmétiques selon la présente demande peuvent aussi se présenter sous forme de mousses.

Par « composition cosmétique coiffante » au sens de la présente demande, on entend une composition pour la mise en forme ou le maintien de la coiffure.

Par « composition liquide » selon la présente demande, on entend que la viscosité de la composition est comprise entre la viscosité de l'eau et 50 poises, de préférence entre la viscosité de l'eau et 20 poises.

Par « composition aqueuse » selon la présente demande, on entend que le milieu cosmétiquement acceptable utilisé dans les compositions selon la présente invention est un milieu hydro-alcoolique ou alcoolique contenant éventuellement au moins un solvant organique additionnel.

L'alcool utilisé dans les compositions selon la présente invention est un alcanol en C1-C4, et de préférence un alcanol monohydroxylé choisi parmi les alcools inférieurs en C₁-C₄ comme l'éthanol, l'isopropanol, le tertiobutanol, le n-butanol, de préférence l'alcool utilisé est l'éthanol.

La concentration en alcool dans les compositions selon la présente invention est comprise entre 0,1 et 99%, de préférence entre 0,5 et 95% et de manière encore plus préférée entre 1 et 80% en poids par rapport au poids total de la composition.

À titre de solvant organique additionnel utilisable dans les compositions selon la présente invention, on compte les polyols comme le propylèneglycol, les éthers de polyols et leurs mélanges.

La concentration en solvant organique additionnel dans les compositions selon la présente invention est comprise entre 0 et 30%, de préférence entre 0 et 20% en poids par rapport au poids total de la composition.

La gomme de gellane est un polysaccharide produit par fermentation aérobie de *Sphingomonas elodea*, plus communément nommé *Pseudomonas elodea*. Ce polysaccharide linéaire est constitué par l'enchaînement des monosaccharides suivants : D-Glucose, acide D-glucuronique et L-Rhamnose. A l'état natif, la gomme de gellane est hautement acylée.

La gomme de gellane utilisée de manière préférée dans les compositions selon la présente invention est une gomme de gellane au moins partiellement déacylée. Cette gomme de gellane au moins partiellement déacylée est obtenue par un traitement alcalin à haute température.

On utilisera par exemple une solution de KOH ou de NaOH.

La gomme de gellane purifiée vendue sous la dénomination commerciale « KELCOGEL® » par la société KELCO convient pour préparer les compositions selon l'invention.

Les dérivés de la gomme de gellane sont tous les produits obtenus en mettant en oeuvre des réactions chimiques classiques, tels que notamment les estérifications, addition d'un sel d'un acide organique ou minéral.

A titre de dérivé de la gomme de gellane, on utilise par exemple la gomme de welane. La gomme de welane est une gomme de gellane modifiée par fermentation au moyen de *Alcaligenes* souche ATCC 31 555. La gomme de welane possède une structure pentasaccharidique récurrente formée d'une chaîne principale constituée d'unités D-Glucose, acide D-glucuronique et L-Rhamnose sur laquelle un motif pendant de L-Rhamnose ou de L-Mannose est greffé.

La gomme de welane vendue sous la dénomination commerciale « KELCO CRETE® » par la société KELCO convient pour préparer les compositions selon l'invention.

La concentration en gomme de gellane ou ses dérivés utilisée dans les compositions selon la présente invention est comprise entre 0,005 et 10%, de préférence entre 0,01 et 5% et de manière encore plus préférée entre 0,02% et 3% en poids par rapport au poids total de la composition.

Les sels monovalents utilisables dans les compositions selon la présente invention sont les sels de cations monovalents tels que les sels des métaux alcalins, les sels d'ammonium, les sels d'amines organiques ou encore leurs mélanges. Les cations monovalents des métaux alcalins sont les cations suivants : Li⁺, Na⁺, K⁺, Rb⁺, Cs⁺, Fr⁺. Dans les compositions selon la présente invention on utilise de préférence le Na⁺.

Le contre-ion est un anion minéral de préférence le contre-ion est Cl⁻.

De préférence, le sel utilisé est NaCl.

La concentration en sel(s) monovalent(s) utilisée dans les compositions selon la présente invention est comprise entre 0,01 et 10%, de préférence entre 0,05 et 5% en poids par rapport au poids total de la composition.

Avantageusement, le rapport sel monovalent/gomme de gellane ou dérivé est compris entre 1 et 50 %, de préférence entre 2 et 30%.

Tous les polymères fixants anioniques, cationiques, amphotères, non ioniques et leurs mélanges utilisés dans la technique peuvent être utilisés dans les compositions selon la présente demande, sont exclus les polymères qui ont uniquement un caractère épaississant tels que les Carbopol ou Carbomer.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 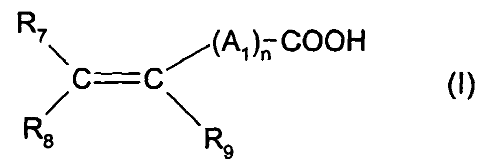 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID et ULTRAHOLD^{®} par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n^{os} 75370 et 75371 ou proposés sous la dénomination QUADRAMER par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF ;
   On peut aussi citer les copolymères acide méthacrylique/ acide acrylique/acrylate d'éthyle/méthacrylate de méthyle en dispersion aqueuse, commercialisé sous la dénomination AMERHOLD^{®} DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ^{®} AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

   Ces polymères sont par exemple décrits dans les brevets français n^{os} 2 350 384 et 2 357 241 de la demanderesse ;
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan^{®} 500 et Flexan^{®} 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL. Comme autre polymère fixant anionique utilisable selon l'invention, on peut citer le polymère anionique séquencé branché vendu sous la dénomination Fixate G-100 par la société NOVEON.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX^{®} A par la société BASF, le polymère vendu sous la dénomination Fixate G-100 par la société NOVEON.

Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF, les terpolymères vinylpyrrolidone/acide acrylique/méthacrylate de lauryle vendus sous la dénomination ACRYLIDONE^{®} LM par la société ISP, le polymère vendu sous la dénomination Fixate G-100 par la société NOVEON.

Les polymères filmogènes fixants cationiques utilisables selon la présente invention sont de préférence choisis parmi les polymères comportant des groupements amines primaires, secondaires, tertiaires et/ou quaternaires faisant partie de la chaîne polymère ou directement reliés à celle-ci, et ayant un poids moléculaire compris entre 500 et environ 5 000 000 et de préférence entre 1 000 et 3 000 000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 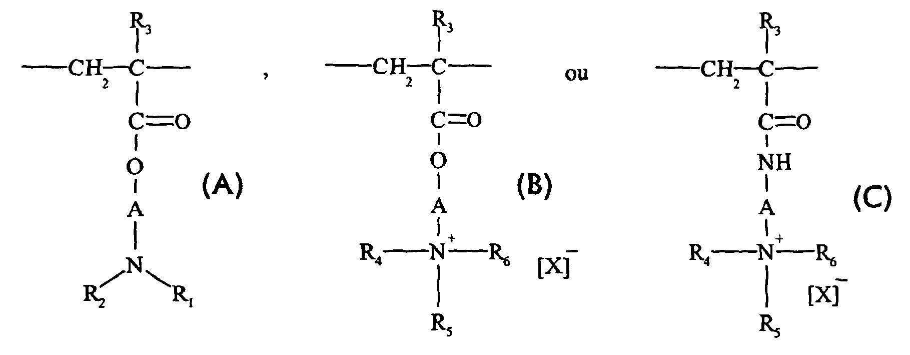 dans lesquelles:
   R₃ désigne un atome d'hydrogène ou un radical CH₃;
   A est un groupe alkyle linéaire ou ramifié comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un radical benzyle;
   R₁ et R₂, identiques ou différents, représentent chacun un atome hydrogène ou un groupe alkyle ayant de 1 à 6 atomes de carbone;
   X désigne un anion méthosulfate ou un halogénure tel que chlorure ou bromure.
      Les copolymères de la famille (1) contiennent en outre un ou plusieurs motifs dérivant de comonomères pouvant être choisis dans la famille des acrylamides, méthacrylamides, diacétones-acrylamides, acrylamides et méthacrylamides substitués sur l'azote par des groupes alkyles inférieurs (C1-C4), des groupes dérivés des acides acryliques ou méthacryliques ou leurs esters, des vinyllactames tels que la vinylpyrrolidone ou le vinylcaprolactame, d'esters vinyliques. Ainsi, parmi ces copolymères de la famille (1), on peut citer :
      - les copolymères d'acrylamide et de méthacrylate de diméthylaminoéthyle quaternisé au sulfate de diméthyle ou avec un halogénure de diméthyle, tels que celui vendu sous la dénomination HERCOFLOC® par la société HERCULES,
      - les copolymères d'acrylamide et de chlorure de méthacryloyloxy-éthyltriméthylammonium décrit par exemple dans la demande de brevet EP-A-080976 et vendus sous la dénomination BINA QUAT P 100 par la société CIBA GEIGY,
      - le copolymère d'acrylamide et de méthosulfate de méthacryloyloxy-éthyltriméthylammonium tel que celui vendu sous la dénomination RETEN par la société HERCULES,
      - les copolymères vinylpyrrolidone/acrylate ou méthacrylate de dialkylaminoalkyle quaternisés ou non, tels que les produits vendus sous la dénomination "GAFQUAT® " par la société ISP comme par exemple "GAFQUAT® 734" ou "GAFQUAT® 755" ou bien les produits dénommés "COPOLYMER® 845, 958 et 937". Ces polymères sont décrits en détail dans les brevets français 2.077.143 et 2.393.573,
      - les polymères à chaîne grasse et à motif vinylpyrrolidone, tels que les produits vendus sous la dénomination Stylèze W20 et Stylèze W10 par la société ISP,
      - les terpolymères méthacrylate de diméthylaminoéthyle/vinylcaprolactame/vinylpyrrolidone tel que le produit commercialisé sous la dénomination GAFFIX VC 713 par la société ISP, et
      - et les copolymères vinylpyrrolidone/méthacrylamide de diméthyl-aminopropyle quaternisés tels que les produits commercialisés sous la dénomination "GAFQUAT® HS 100" par la société ISP.
   (2) les polysaccharides cationiques, de préférence à ammonium quaternaire tels que ceux décrits dans les brevets américains 3.589.578 et 4.031.307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL.
   (3) les copolymères quaternaires de vinylpyrrolidone et de vinylimidazole ;
   (4) les chitosanes ou leurs sels; les sels utilisables sont en particulier les acétate, lactate, glutamate, gluconate ou le pyrrolidone-carboxylate de chitosane .
      Parmi ces composés, on peut citer le chitosane ayant un taux de désacétylation de 90,5% en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL .
   (5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amines primaires ou secondaires.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkyl-méthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les composés dont les groupes alkyles comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER^{®} ou LOVOCRYL^{®} 47 par la société NATIONAL STARCH.
(3) les polyaminoamides réticulés et acylés partiellement ou totalement dérivant de polyaminoamides de formule générale : 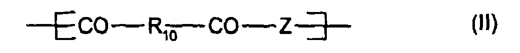 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 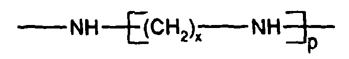 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IV) ci-dessus, dans lequel x=2 et p=1 et qui dérive de l'éthylène-diamine, ou le groupe dérivant de la pipérazine : 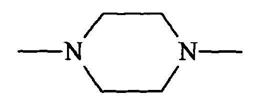
   c) dans les proportions de 0 à 20 % en moles, le groupe -NH-(CH₂)₆-NH- dérivant de l'hexaméthylènediamine, ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 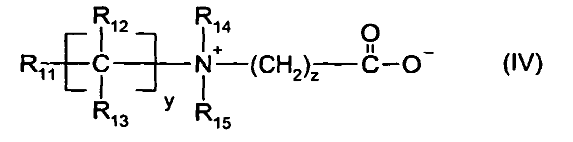 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle, tel que le produit vendu sous la dénomination DIAFORMER Z301 par la société SANDOZ.
(5) les polymères dérivés du chitosane comportant des motifs monomères répondant aux formules suivantes : 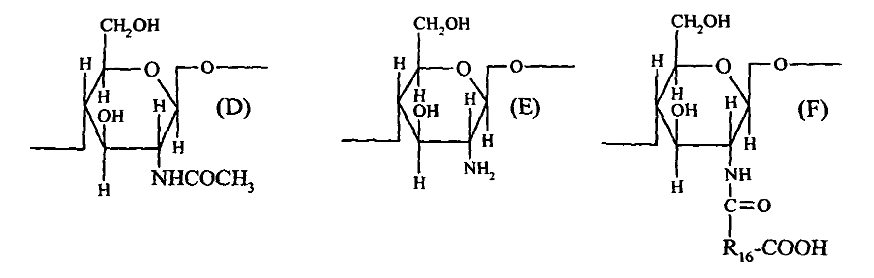 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 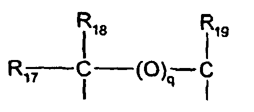 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 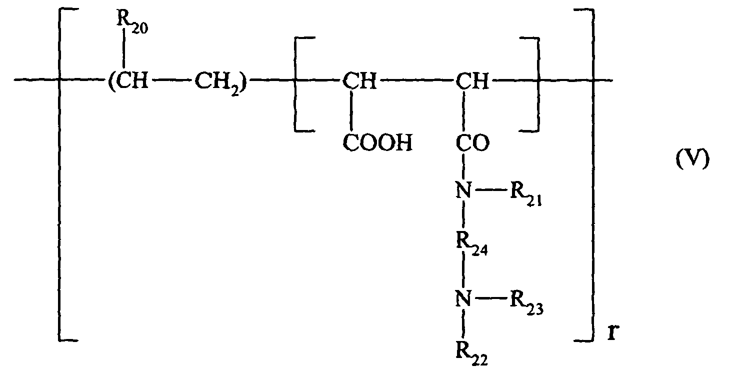 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂- , -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutyl-chitosane, vendus sous la dénomination « EVALSAN » par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 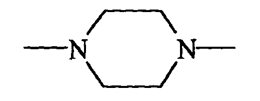 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')
   où D désigne un groupe 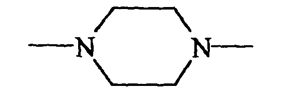 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyles et comportant un ou plusieurs atomes d'azote, l'atome d'azote étant substitué par une chaîne alkyle interrompue éventuellement par un atome d'oxygène et comportant obligatoirement une ou plusieurs fonctions carboxyles ou une ou plusieurs fonctions hydroxyles et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine ou par semiestérification avec un N,N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER^{®}, AMPHOMER^{®} LV 71 ou LOVOCRYL^{®} 47 par la société NATIONAL STARCH et ceux de la famille (4) tels que les copolymères méthacrylate de méthyle/diméthyl-carboxyméthylammonio-éthylméthacrylate de méthyle vendu par exemple sous la dénomination DIAFORMER Z301 par la société SANDOZ.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyles des polymères non ioniques mentionnés ci-dessus ont, de préférence, de 1 à 6 atomes de carbone.

Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 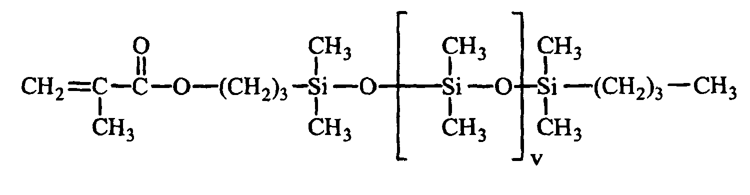
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex^{®} Silk commercialisé par la société BASF.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR^{®} et LUVISET^{®} Si PUR par la société BASF.

La concentration en polymère(s) fixant(s) utilisée dans les compositions selon la présente invention est comprise entre 0,1 et 20%, de préférence entre 0,5 et 10% en poids par rapport au poids total de la composition.

La composition coiffante selon l'invention peut contenir en outre au moins un adjuvant choisi parmi les silicones sous forme soluble, dispersée, micro-dispersée, les agents tensio-actifs non-ioniques, anioniques, cationiques et amphotères, les polymères non-ioniques, anioniques, cationiques et amphotères additionnels autres que les polymères fixants utilisés dans les compositions selon l'invention, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, les particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

L'homme de métier veillera à choisir les éventuels additifs et leur quantité de manière à ce qu'ils ne nuisent pas aux propriétés des compositions selon la présente invention.

Ces additifs sont présents dans la composition selon l'invention en une quantité allant de 0 à 20 % en poids par rapport au poids total de la composition.

Les exemples suivants illustrent la présente invention et ne doivent être considérés en aucune manière comme limitant l'invention.

### EXEMPLE

On prépare une composition formulée en spray en flacon pompe dont les teneurs des différents constituants en g/100g sont les suivantes :

| Constituant | g/100g |
|---|---|
| UltraHold® Strong commercialisé par BASF | 2 |
| AMP(aminométhylpropanol) | 0,2 |
| Gomme de gellane | 1 |
| NaCl | 2 |
| Paillettes (Mica et Oxyde de titane) | 0,5 |
| Eau distillée | 45 |
| Parfum | qs |
| Alcool | Qsp 100 |

UltraHold® Strong commercialisé par BASF est un terpolymère fixant anionique d'acide acrylique/acrylate d'éthyle/N-tertiobutylacrylamide.

## Revendications

1. Composition cosmétique liquide, aqueuse, contenant, dans un milieu cosmétiquement acceptable au moins une gomme de gellane ou un de ses dérivés, au moins un polymère fixant, au moins un sel de cation monovalent, le centre-ion étant un anion minéral, et au moins un alcool choisi parmi les alcanols en C1-C4.

2. Composition cosmétique selon la revendication 1 telle que le dérivé de gomme de gellane est une gomme de welane.

3. Composition cosmétique selon la revendication 1 ou 2 telle que la concentration en gomme de gellane ou ses dérivés est comprise entre 0,005 et 10%, de préférence entre 0,01 et 5% et de manière encore plus préférée entre 0,02% et 3% en poids par rapport au poids total de la composition.

4. Composition cosmétique selon l'une des revendications précédentes telle que le polymère fixant est choisi parmi les polymères fixants anioniques, cationiques, amphotères, non-ioniques, ou leurs mélanges.

5. Composition selon la revendication 4, **caractérisée en ce que** le polymère fixant cationique est choisi parmi les homopolymères ou copolymères d'esters ou d'amides acryliques ou méthacryliques à fonctions aminées, les polysaccharides cationiques, les copolymères quaternaires de vinylpyrrolidone et de vinylimidazole, et les chitosanes.

6. Composition selon la revendication 4, **caractérisée en ce que** le polymère fixant anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, les polyuréthanes anioniques, et les polymères siliconés greffés anioniques.

7. Composition selon la revendication 4, **caractérisée en ce que** le polymère fixant amphotère est choisi parmi les copolymères à motifs vinyliques acides et à motifs vinyliques basiques, les polyaminoamides réticulés et acylés, les polymères à motifs zwittérioniques, les polymères dérivés du chitosane, les copolymères alkyl(C₁₋C₅)vinyléther/anhydride maléique modifiés, les polyuréthanes amphotères et les polymères siliconés greffés amphotères.

8. Composition selon la revendication 4, **caractérisée en ce que** le polymère fixant non ionique est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame, les polyuréthanes non ioniques, et les polymères siliconés greffés non ioniques.

9. Composition cosmétique selon l'une des revendications 4 à 8 telle que la concentration en polymère(s) fixant(s) est comprise entre 0,1 et 20%, de préférence entre 0,5 et 10% en poids par rapport au poids total de la composition.

10. Composition cosmétique selon l'une des revendications précédentes telle que le sel de cation monovalent est choisi parmi les sels des métaux alcalins, les sels d'ammonium, les sels d'amines organiques ou encore leurs mélanges.

11. Composition cosmétique selon la revendication 10 telle que le sel monovalent de métal alcalin est un sel de Na⁺.

12. Composition cosmétique selon l'une des revendications précédentes telle que la concentration en sel monovalent est comprise entre 0,01 et 10%, de préférence entre 0,05 et 5% en poids par rapport au poids total de la composition.

13. Composition cosmétique selon l'une des revendications précédentes telle que l'alcool est choisi parmi l'éthanol, l'isopropanol, le tertiobutanol, le n-butanol.

14. Composition cosmétique selon la revendication 13 telle que l'alcool est l'éthanol.

15. Composition cosmétique selon l'une des revendications précédentes telle que la concentration en alcool est comprise entre 0,1 et 99%, de préférence entre 0,5 et 95% en poids par rapport au poids total de la composition.

16. Composition cosmétique selon l'une des revendications précédentes telle qu'elle contient au moins un adjuvant choisi parmi les silicones sous forme soluble, dispersée, micro-dispersée, les agents tensio-actifs non-ioniques, anioniques, cationiques et amphotères, les polymères non-ioniques, anioniques, cationiques et amphotères additionnels, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, les particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

17. Composition cosmétique selon l'une des revendications précédentes telle qu'elle se présente sous la forme d'un spray.

18. Cosmétique selon l'une des revendications 1 à 16 telle qu'elle se présente sous la forme d'un gel.

19. Cosmétique selon l'une des revendications 1 à 16 telle qu'elle se présente sous la forme d'une mousse.

20. Procédé pour la mise en forme ou le maintien de la coiffure dans lequel la composition cosmétique selon l'une des revendications 1 à 19 est mise en oeuvre

21. Utilisation de la composition cosmétique selon l'une des revendications 1 à 19 en tant que composition coiffante pour la fixation et le maintien des cheveux.

22. Utilisation de la composition cosmétique selon l'une des revendications 1 à 19 en tant que composition de soin des cheveux.

23. Utilisation de la composition cosmétique selon l'une des revendications 1 à 19 en tant que composition de conditionnement des cheveux notamment pour apporter de la douceur aux cheveux.

24. Utilisation de la composition cosmétique selon l'une des revendications 1 à 19 en tant que composition de maquillage des cheveux.

## Claims

1. Aqueous liquid cosmetic composition containing, in a cosmetically acceptable medium, at least one gellan gum or a derivative thereof, at least one fixing polymer, at least one salt having a monovalent cation, the counter ion being a mineral anion, and at least one alcohol chosen from C₁-C₄ alkanols.

2. Cosmetic composition according to Claim 1, such that the gellan gum derivative is a welan gum.

3. Cosmetic composition according to Claim 1 or 2, such that the concentration of gellan gum or derivatives thereof is between 0.005% and 10%, preferably between 0.01% and 5% and even more preferably between 0.02% and 3% by weight relative to the total weight of the composition.

4. Cosmetic composition according to one of the preceding claims, such that the fixing polymer is chosen from anionic, cationic, amphoteric and nonionic fixing polymers, or mixtures thereof.

5. Composition according to Claim 4, **characterized in that** the cationic fixing polymer is chosen from homopolymers or copolymers of acrylic or methacrylic esters or amides containing amine functions, cationic polysaccharides, quaternary copolymers of vinylpyrrolidone and of vinylimidazole, and chitosans.

6. Composition according to Claim 4, **characterized in that** the anionic fixing polymer is chosen from acrylic or methacrylic acid homopolymers or copolymers or salts thereof, crotonic acid copolymers, copolymers of C4-C8 monounsaturated carboxylic acids or anhydrides, polyacrylamides containing carboxylate groups, homopolymers or copolymers containing sulfonic groups, anionic polyurethanes and anionic grafted silicone polymers.

7. Composition according to Claim 4, **characterized in that** the amphoteric fixing polymer is chosen from copolymers containing acidic vinyl units and basic vinyl units, crosslinked and acylated polyamino amides, polymers containing zwitterionic units, chitosan-based polymers, (C1-C5)alkyl vinyl ether/maleic anhydride modified copolymers, amphoteric polyurethanes and amphoteric grafted silicone polymers.

8. Composition according to Claim 4, **characterized in that** the nonionic fixing polymer is chosen from polyalkyloxazolines, vinyl acetate homopolymers and copolymers, acrylic ester homopolymers and copolymers, acrylonitrile copolymers, styrene homopolymers and copolymers, polyamides, vinyllactam homopolymers other than vinylpyrrolidone homopolymers, vinyllactam copolymers, nonionic polyurethanes and nonionic grafted silicone polymers.

9. Cosmetic composition according to one of Claims 4 to 8, such that the concentration of fixing polymer(s) is between 0.1% and 20% and preferably between 0.5% and 10% by weight relative to the total weight of the composition.

10. Cosmetic composition according to one of the preceding claims, such that the salt having a monovalent cation is chosen from alkali metal salts, ammonium salts and organic amine salts, or mixtures thereof.

11. Cosmetic composition according to Claim 10, such that the monovalent alkali metal salt is an Na⁺ salt.

12. Cosmetic composition according to one of the preceding claims, such that the concentration of monovalent salt is between 0.01% and 10% and preferably between 0.05% and 5% by weight relative to the total weight of the composition.

13. Cosmetic composition according to one of the preceding claims, such that the alcohol is chosen from ethanol, isopropanol, tert-butanol or n-butanol.

14. Cosmetic composition according to Claim 13, such that the alcohol is ethanol.

15. Cosmetic composition according to one of the preceding claims, such that the alcohol concentration is between 0.1% and 99% and preferably between 0.5% and 95% by weight relative to the total weight of the composition.

16. Cosmetic composition according to one of the preceding claims, such that it contains at least one adjuvant chosen from silicones in soluble, dispersed or microdispersed form, nonionic, anionic, cationic and amphoteric surfactants, nonionic, anionic, cationic and amphoteric additional polymers, ceramides, pseudoceramides, vitamins and provitamins, including panthenol, plant, animal, mineral and synthetic oils, waxes other than ceramides and pseudoceramides, silicone-based or non-silicone-based water-soluble and liposoluble sunscreens, solid particles, for instance coloured or uncoloured, mineral and organic pigments, nacreous agents and opacifiers, flakes, active particles, dyes, sequestrants, plasticizers, solubilizers, acidifying agents, basifying agents, neutralizers, mineral and organic thickeners, antioxidants, hydroxy acids, penetrants, fragrances and preserving agents.

17. Cosmetic composition according to one of the preceding claims, such that it is in the form of a spray.

18. Cosmetic composition according to one of Claims 1 to 16, such that it is in the form of a gel.

19. Cosmetic composition according to one of Claims 1 to 16, such that it is in the form of a mousse.

20. Process for shaping or holding the hairstyle, in which the cosmetic composition according to one of Claims 1 to 19 is used.

21. Use of the cosmetic composition according to one of Claims 1 to 19 as a styling composition for fixing and holding the hair.

22. Use of the cosmetic composition according to one of Claims 1 to 19, as a haircare composition.

23. Use of the cosmetic composition according to one of Claims 1 to 19, as a hair conditioning composition, especially for giving the hair softness.

24. Use of the cosmetic composition according to one of Claims 1 to 19, as a hair makeup composition.

## Patentansprüche

1. Flüssige, wässrige kosmetische Zusammensetzung, die in einem kosmetisch akzeptablen Medium mindestens ein Gellan oder eines seiner Derivate, mindestens ein Salz eines einwertigen Kations, wobei das Gegenion in anorganisches Anion ist, mindestens ein festigendes Polymer und mindestens einen Alkohol enthält, der unter den C₁₋₄-Alkanolen ausgewählt ist.

2. Kosmetische Zusammensetzung nach Anspruch 1, wobei das Gellanderivat ein Welan-Gummi ist.

3. Kosmetische Zusammensetzung nach Anspruch 1 oder 2, wobei die Konzentration des Gellans oder seiner Derivate im Bereich von 0,005 bis 10 Gew.-%, vorzugsweise 0,01 bis 5 Gew.-% und noch bevorzugter im Bereich von 0,02 bis 3 Gew.-%, bezogen auf das Gesamtgewicht der Zusammensetzung, liegt.

4. Kosmetische Zusammensetzung nach einem der vorhergehenden Ansprüche, wobei das festigende Polymer unter den anionischen, kationischen, amphoteren und nichtionischen festigenden Polymeren oder deren Gemischen ausgewählt ist.

5. Zusammensetzung nach Anspruch 4, **dadurch gekennzeichnet, dass** das kationische festigende Polymer unter den Homopolymeren oder Copolymeren von Acrylestern, Methacrylestern, Acrylamiden oder Methacrylamiden mit aminierten Funktionen, kationischen Polysacchariden, quartären Copolymeren von Vinylpyrrolidon und Vinylimidazol und Chitosanen ausgewählt ist.

6. Zusammensetzung nach Anspruch 4, **dadurch gekennzeichnet, dass** das anionische festigende Polymer unter den Homopolymeren oder Copolymeren von Acrylsäure und Methacrylsäure oder deren Salzen, Copolymeren von Crotonsäure, Copolymeren von einfach ungesättigten Carbonsäuren oder Carbonsäureanhydriden mit 4 bis 8 Kohlenstoffatomen, Polyacrylamiden mit Carboxylatgruppen, Homopolymeren oder Copolymeren mit Sulfonsäuregruppen, anionischen Polyurethanen und anionischen gepfropften Siliconpolymeren ausgewählt ist.

7. Zusammensetzung nach Anspruch 4, **dadurch gekennzeichnet, dass** das amphotere festigende Polymer unter den Copolymeren mit sauren Vinyleinheiten und mit basischen Vinyleinheiten, vernetzten und acylierten Polyaminoamiden, Polymeren mit zwitterionischen Einheiten, von Chitosan abgeleiteten Polymeren, modifizierten Alkyl(C₁₋₅)vinylether/Maleinsäureanhydrid-Copolymeren, amphoteren Polyurethanen und amphoteren gepfropften Siliconpolymeren ausgewählt ist.

8. Zusammensetzung nach Anspruch 4, **dadurch gekennzeichnet, dass** das nichtionische festigende Polymer unter den Polyalkyloxazolinen, Homopolymeren und Copolymeren von Vinylacetat, Homopolymeren und Copolymeren von Acrylestern, Copolymeren von Acrylnitril, Homopolymeren und Copolymeren von Styrol, Polyamiden, Homopolymeren von Vinyllactam, die von den Homopolymeren von Vinylpyrrolidon verschieden sind, Copolymeren von Vinyllactam, nichtionischen Polyurethanen und nichtionischen gepfropften Siliconpolymeren ausgewählt ist.

9. Kosmetische Zusammensetzung nach einem der Ansprüche 4 bis 8, wobei die Konzentration des festigenden Polymers (der festigenden Polymere) im Bereich von 0,1 bis 20 Gew.-% und vorzugsweise 0,5 bis 10 Gew.-%, bezogen auf das Gesamtgewicht der Zusammensetzung, liegt.

10. Kosmetische Zusammensetzung nach einem der vorhergehenden Ansprüche, wobei das Salz des einwertigen Kations unter den Salzen von Alkalimetallen, Ammoniumsalzen, Salzen von organischen Aminen oder deren Gemischen ausgewählt ist.

11. Kosmetische Zusammensetzung nach Anspruch 10, wobei das einwertige Alkalimetallsalz ein Na⁺-Salz ist.

12. Kosmetische Zusammensetzung nach einem der vorhergehenden Ansprüche, wobei die Konzentration des einwertigen Salzes im Bereich von 0,01 bis 10 Gew.-% und vorzugsweise 0,05 bis 5 Gew.-%, bezogen auf das Gesamtgewicht der Zusammensetzung, liegt.

13. Kosmetische Zusammensetzung nach einem der vorhergehenden Ansprüche, wobei der Alkohol unter Ethanol, Isopropanol, *tert*-Butanol und *n*-Butanol ausgewählt ist.

14. Kosmetische Zusammensetzung nach Anspruch 13, wobei es sich bei dem Alkohol um Ethanol handelt.

15. Kosmetische Zusammensetzung nach einem der vorhergehenden Ansprüche, wobei die Konzentration des Alkohols im Bereich von 0,1 bis 99 Gew.-% und vorzugsweise 0,5 bis 95 Gew.-%, bezogen auf das Gesamtgewicht der Zusammensetzung, liegt.

16. Kosmetische Zusammensetzung nach einem der vorhergehenden Ansprüche, wobei sie mindestens einen Zusatzstoff enthält, der unter den in solubilisierter, dispergierter oder mikrodispergierter Form vorliegenden Siliconen, nichtionischen, anionischen, kationischen und amphoteren grenzflächenaktiven Stoffen, ergänzenden nichtionischen, anionischen, kationischen und amphoteren Polymeren, Ceramiden und Pseudoceramiden, Vitaminen und Provitaminen, darunter Panthenol, pflanzlichen Ölen, tierischen Ölen, Mineralölen und synthetischen Ölen, Wachsen, die von den Ceramiden und Pseudoceramiden verschieden sind, wasserlöslichen und fettlöslichen Sonnenschutzfiltern, die siliconiert oder nichtsiliconiert sind, festen Partikeln, wie beispielsweise farbigen oder nichtfarbigen, anorganischen und organischen Pigmenten, Perglanzmitteln und Trübungsmitteln, Pailletten, Aktivpartikeln, Farbmitteln, Maskierungsmitteln, Weichmachern, Solubilisierungsmitteln, Ansäuerungsmitteln, Alkalisierungsmitteln, Neutralisationsmitteln, anorganischen und organischen Verdickungsmitteln, Antioxidantien, Hydroxysäuren, Penetrationsmitteln, Parfums und Konservierungsmitteln ausgewählt ist.

17. Kosmetische Zusammensetzung nach einem der vorhergehenden Ansprüche, wobei sie als Spray vorliegt.

18. Kosmetische Zusammensetzung nach einem der Ansprüche 1 bis 16, wobei sie als Gel vorliegt.

19. Kosmetische Zusammensetzung nach einem der Ansprüche 1 bis 16, wobei sie als Mousse vorliegt.

20. Verfahren für die Formgebung oder Festigung der Frisur, wobei eine kosmetische Zusammensetzung nach einem der Ansprüche 1 bis 19 verwendet wird.

21. Verwendung der kosmetischen Zusammensetzung nach einem der Ansprüche 1 bis 19 als Zusammensetzung für die Formgebung und Festigung der Frisur.

22. Verwendung der kosmetischen Zusammensetzung nach einem der Ansprüche 1 bis 19 als Zusammensetzung für die Pflege der Haare.

23. Verwendung der kosmetischen Zusammensetzung nach einem der Ansprüche 1 bis 19 als Zusammensetzung für die Konditionierung der Haare, insbesondere um den Haaren Weichheit zu geben.

24. Verwendung der kosmetischen Zusammensetzung nach einem der Ansprüche 1 bis 19 als Zusammensetzung zum Schminken der Haare.
